(19) Europäisches Patentamt
European Patent Office
Office européen des brevets

(11) Publication number: **0 276 163 B1**

(12) # EUROPEAN PATENT SPECIFICATION

(45) Date of publication of patent specification: **15.07.92**

(51) Int. Cl.[5]: **C07D 403/06**, A61K 31/415

(21) Application number: **88300522.5**

(22) Date of filing: **22.01.88**

(54) **Indole derivatives.**

(30) Priority: **23.01.87 GB 8701494**

(43) Date of publication of application:
**27.07.88 Bulletin 88/30**

(45) Publication of the grant of the patent:
**15.07.92 Bulletin 92/29**

(84) Designated Contracting States:
**AT BE CH DE ES FR GB GR IT LI LU NL SE**

(56) References cited:
**EP-A- 0 003 901**
**EP-A- 0 191 562**
**EP-A- 0 242 973**
**US-A- 3 459 770**

(73) Proprietor: **GLAXO GROUP LIMITED**
**Clarges House 6-12 Clarges Street**
**London W1Y 8DH(GB)**

(72) Inventor: **Oxford, Alexander William**
**60 Green Drift**
**Royston Hertfordshire(GB)**
Inventor: **Cavalla, David John**
**20 Thistlewaite Road**
**London E5 0OO(GB)**

(74) Representative: **Marchant, James Ian et al**
**Elkington and Fife Prospect House 8 Pembroke Road**
**Sevenoaks, Kent TN13 1XR(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to indole derivatives, to processes for their preparation, to pharmaceutical compositions containing them and to their medical use. In particular the invention relates to compounds which act upon 5-hydroxytryptamine (5-HT) receptors of the type located on terminals of primary afferent nerves.

Compounds having antagonist activity at 'neuronal' 5-HT receptors of the type located on primary afferent nerves have been described previously.

Thus for example published UK Patent Specification No. 2153821A and published European Patent Specification No. 191562 disclose tetrahydrocarbazolones of the general formula

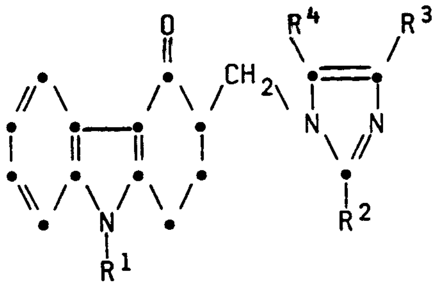

wherein $R^1$ represents a hydrogen atom or a $C_{1-10}$alkyl, $C_{3-7}$cycloalkyl, $C_{3-7}$cycloalkyl$C_{1-4}$alkyl, $C_{3-6}$alkenyl, $C_{3-10}$alkynyl, phenyl or phenyl$C_{1-3}$alkyl group, and one of the groups represented by $R^2$, $R^3$ and $R^4$ is a hydrogen atom or a $C_{1-6}$alkyl, $C_{3-7}$cycloalkyl, $C_{2-6}$alkenyl or phenyl$C_{1-3}$alkyl group and each of the other two groups, which may be the same or different, represents a hydrogen atom or a $C_{1-6}$ alkyl group.

We have now found a novel group of compounds which differ in structure from those described previously, and which are potent antagonists of the effect of 5-HT at 5-HT 'neuronal' receptors.

Thus, in one aspect the present invention provides an indole of the general formula (I):

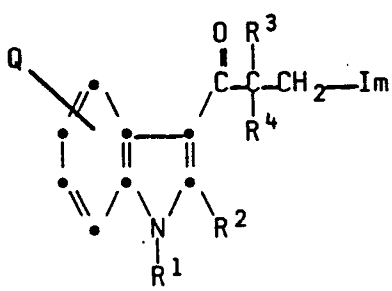

(I)

wherein Im represents an imidazolyl group of formula:

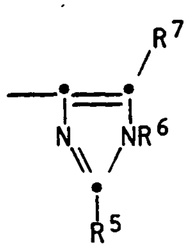

or

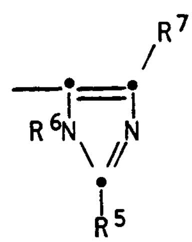

$R^1$ represents a hydrogen atom or a group selected from $C_{1-6}$alkyl, $C_{3-6}$ alkenyl, $C_{3-10}$ alkynyl, $C_{3-7}$ cycloalkyl, $C_{3-7}$cycloalkyl$C_{1-4}$alkyl, phenyl, phenyl$C_{1-3}$alkyl, $-CO_2R^8$, $-COR^8$, $-CONR^8R^9$ or $-SO_2R^8$ (wherein $R^8$ and $R^9$, which may be the same of different, each represents a hydrogen atom, a $C_{1-6}$ alkyl or $C_{3-7}$ cycloalkyl group, or a phenyl or phenyl$C_{1-4}$alkyl group, in which the phenyl group is optionally substituted

by one or more $C_{1-4}$ alkyl, $C_{1-4}$ alkoxy or hydroxy groups or halogen atoms, with the proviso that $R^8$ does not represent a hydrogen atom when $R^1$ represents a group $-CO_2R^8$ or $-SO_2R^8$);
$R_2$ represents a hydrogen atom or a $C_{1-6}$ alkyl, $C_{3-6}$ alkenyl, $C_{3-7}$ cycloalkyl, phenyl or phenyl$C_{1-3}$alkyl group;
$R^3$ and $R^4$, which may be the same or different, each represents a hydrogen atom or a $C_{1-6}$ alkyl group;
one of the groups represented by $R^5$, $R^6$ and $R^7$ is a hydrogen atom or a $C_{1-6}$ alkyl, $C_{3-7}$ cycloalkyl, $C_{3-6}$ alkenyl, phenyl or phenyl$C_{1-3}$alkyl group, and each of the other two groups, which may be the same or different, represents a hydrogen atom or a $C_{1-6}$ alkyl group;
Q represents a hydrogen atom or a halogen atom or a hydroxy, $C_{1-4}$alkoxy, phenyl$C_{1-3}$alkoxy or $C_{1-6}$ alkyl group or a group $-NR^{10}R^{11}$ or $-CONR^{10}R^{11}$ (wherein $R^{10}$ and $R^{11}$, which may be the same or different, each represents a hydrogen atom or a $C_{1-4}$ alkyl or $C_{3-4}$alkenyl group, or together with the nitrogen atom to which they are attached form a saturated 5 to 7 membered ring); and with the proviso that when Q represents a hydrogen atom $R^1$ represents $-CO_2R^8$, $-COR^8$, $-CONR^8R^9$ or $-SO_2R^8$; and physiologically acceptable salts and solvates thereof.

Suitable physiologically acceptable salts of the compounds of general formula (I) include acid addition salts formed with organic or inorganic acids for axample, hydrochlorides, hydrobromides, sulphates, alkyl- or arylsulphonates (e.g. methanesulphonates or p-toluenesulphonates), phosphates, acetates, citrates, succinates, tartrates, fumarates and maleates. The solvates may, for example, be hydrates.

All optical isomers of compounds of general formula (I) and their mixtures including the racemic mixtures thereof, and all the geometric isomers of compounds of formula (I), are embraced by the invention.

It will be appreciated that the invention extends to other physiologically acceptable equivalents of the compounds according to the invention, i.e. physiologically acceptable compounds which are converted in vivo into the parent compound of formula (I).

Referring to the general formula (I), the alkyl groups represented by $R^1$, $R^2$, $R^3$, $R^4$, $R^5$, $R^6$, $R^7$, $R^8$, $R^9$, $R^{10}$, $R^{11}$ and Q may be straight chain or branched chain alkyl groups, for example, methyl, ethyl, propyl, prop-2-yl, butyl, but-2-yl or 2-methylprop-2-yl, and, in the case of $R^1$-$R^9$ and Q, pentyl, pent-3-yl or hexyl. An alkenyl group may be, for example, a propenyl or butenyl group. An alkynyl group may be, for example, a prop-2-ynyl or oct-2-ynyl group.

When $R^1$ represents a $C_{3-6}$ alkenyl group or a $C_{3-10}$ alkynyl group, or $R^{10}$ or $R^{11}$ represents a $C_{3-4}$alkenyl group, the double or triple bond may not be adjacent to the nitrogen atom.

A phenyl$C_{1-3}$ alkyl group (as such or as part of a phenyl$C_{1-3}$alkoxy group) may be, for example, a benzyl, phenethyl or 3-phenylpropyl group. A cycloalkyl group (as such or as part of a cycloalkylalkyl group) may be, for example, a cyclopropyl, cyclobutyl, cyclopentyl, cyclohexyl or cycloheptyl group. When $R^1$ represents a $C_{3-7}$cycloalkyl$C_{1-4}$alkyl group, the alkyl moiety may be, for example, a methyl, ethyl, propyl, prop-2-yl or butyl group. When Q represents a $C_{1-4}$ alkoxy group it may be, for example, a methoxy group. When Q represents a halogen atom it may be, for example a fluorine, chlorine bromine atom. The substituent Q may be at the 4, 5, 6 or 7 position of the indole moiety.

A preferred class of compounds of formula (I) are those where $R^1$ represents a hydrogen atom or a $C_{1-3}$ alkyl, $C_{3-4}$ alkenyl, $C_{3-4}$ alkynyl, $C_{5-6}$cycloalkyl, benzyl, N,N-di$C_{1-3}$alkylcarboxamido or $C_{1-3}$alkoxycarbonyl group. More preferably $R^1$ represents a $C_{1-3}$ alkyl (e.g. methyl), N,N-di$C_{1-3}$alkylcarboxamido (e.g. N,N-dimethylcarboxamido) or $C_{1-3}$ alkoxycarbonyl (e.g. methoxycarbonyl group.

Another preferred class of compounds of formula (I) are those wherein $R^2$ represents a hydrogen atom or a $C_{1-3}$ alkyl (e.g. methyl) group, more preferably a hydrogen atom.

Another preferred class of compounds of formula (I) are those wherein $R^3$ and $R^4$ each independently represent a hydrogen atom or a $C_{1-3}$ alkyl (e.g. methyl) group. A particularly preferred class of compounds are those in which $R^3$ and $R^4$ both represent hydrogen atoms.

A further preferred class of compounds of formula (I) are those wherein $R^5$ represents a $C_{1-3}$ alkyl (e.g. methyl) group or, more preferably, a hydrogen atom. $R^6$ preferably represents a $C_{1-3}$ alkyl (e.g. methyl) or, more preferably, a hydrogen atom. $R^7$ preferably represents a hydrogen atom or a $C_{1-3}$ alkyl (e.g. methyl) group. When $R^5$ and $R^6$ represent hydrogen atoms, $R^7$ is preferably a $C_{1-6}$ alkyl, $C_{3-7}$ cycloalkyl, $C_{3-6}$ alkenyl, or phenyl$C_{1-3}$ alkyl group, more particularly $C_{1-3}$ alkyl (e.g. methyl).

Another preferred class of compounds of formula (I) are those wherein Q represents a hydrogen atom, a halogen (e.g. fluorine) atom or a hydroxy, $C_{1-3}$alkoxy (e.g. methoxy) or $C_{1-3}$alkyl (e.g. methyl) group. More preferably Q represents a hydrogen or halogen (e.g. fluorine) atom or a $C_{1-3}$alkyl (e.g. methyl) group.

When Q is other than a hydrogen atom, it is preferably at the 5-, 6- or 7-position of the indole ring, more preferably the 5- or 7-position.

A preferred group of compounds of formula (I) are those wherein $R^1$ represents a $C_{1-3}$ alkyl (e.g. methyl), N,N-di$C_{1-3}$alkylcarboxamido (e.g. N,N-dimethylcarboxamido) or $C_{1-3}$alkoxycarbonyl (e.g. methoxycarbonyl) group, more preferably a methyl group; $R^2$ represents a hydrogen atom; $R^3$ and $R^4$ both

represent hydrogen atoms; $R^5$ and $R^6$ each represent a hydrogen atom; $R^7$ represents a hydrogen atom or a $C_{1-3}$alkyl group, more preferably a methyl group; and Q represents a hydrogen or halogen (e.g. fluorine) atom or a $C_{1-3}$ alkyl (e.g. methyl) group.

A particularly preferred compound according to the invention is 3-(5-methyl-1H-imidazol-4-yl)-1-(1,7-dimethyl-1H-indol-3-yl)-propanone and its physiologically acceptable salts and solvates.

Compounds of the invention are potent and selective antagonists or 5-HT-induced responses of the rat isolated vagus nerve preparation and thus act as potent and selective antagonists of the 'neuronal' 5-HT receptor type located on primary afferent nerves. Receptors of this type are now designated as 5-$HT_3$ receptors. Such receptors are also present in the central nervous system. 5-HT occurs widely in the neuronal pathways in the central nervous system and disturbance of these 5-HT containing pathways is known to alter behavioural syndromes such as mood, psychomotor activity, appetite and memory.

Compounds of formula (I), which antagonise the effect of 5-HT at 5-$HT_3$ receptors, are useful in the treatment of conditions such as psychotic disorders (e.g. schizophrenia and mania); anxiety; and nausea and vomiting, particularly that associated with cancer chemotherapy and radiotherapy. Compounds of formula (I) are also useful in the treatment of gastric stasis; symptoms of gastrointestinal dysfunction such as occur with dyspepsia, peptic ulcer, reflux oesophagitis, flatulence and irritable bowel syndrome; migraine; and pain. Compounds of formula (I) may also be used in the treatment of dependency on drugs and substances of abuse, depression, and dementia and other cognitive disorders.

Unlike existing drug treatments for certain of the above conditions, the compounds of the invention, because of their high selectivity for 5-$HT_3$ receptors, would not be expected to produce undesirable side effects. Thus, for example, neuroleptic drugs may cause extrapyramidal effects, such as tardive dyskinesia, and benzodiazepines may cause dependence.

According to another aspect, the invention provides a method of treatment of a human or animal subject suffering from a psychotic disorder such as schizophrenia or mania; or from anxiety; nausea or vomiting, particularly that associated with cancer chemotherapy and radiotherapy; gastric stasis; symptoms of gastrointestinal dysfunction such as dyspepsia, reflux oesophagitis, peptic ulcer, flatulence oand irritable bowel syndrome; migraine; pain; dependency on drugs or substances of abuse; depression; or dementia and other cognitive disorders, which comprises administering an effective amount of a compound of formula (I) or a physiologically acceptable salt or solvate thereof.

Accordingly, the invention also provides a pharmaceutical composition which comprises at least one compound selected from indole derivatives of the general formula (I), their physiologically acceptable salts and solvates (e.g. hydrates), for use in human or veterinary medicine, and formulated for administration by any convenient route.

Such compositions may be formulated in conventional manner using one or more physiologically acceptable carriers and/or excipients.

Thus the compounds according to the invention may be formulated for oral, buccal, parenteral, rectal or transdermal administration or in a form suitable for administration by inhalation or insufflation (either through the mouth or the nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by injection e.g. by bolus injection or continuous infusion. Formulations for injection may be presented in unit dosage form e.g. in ampoules or in multi-dose containers, with an added preservative. The compositions may take such

forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described prviously, the compounds of the invention may also be formulated as depot preparations. Such long acting formulations may be administered by implantation (for example subcutaneously, transcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

For intranasal administration, the compounds according to the invention may be formulated as solutions for administration via a suitable metered or unit dose device or alternatively as a powder mix with a suitable carrier for administration using a suitable delivery device.

The compounds of formula (I) may also be administered in combination with other therapeutic agents. Thus, for example, in the treatment of gastric stasis, symptoms of gastrointestinal dysfunction and nausea and vomiting, the compounds of formula (I) may be administered in combination with antisecretory agents such as histamine $H_2$-receptor antagonists (e.g. ranitidine, sufotidine or loxtidine) or $H^+K^+$ATPase inhibitors (e.g. omeprazole).

A proposed dose of the compounds of the invention for administration to man (of approximately 70kg body weight) is 0.001 to 100mg, preferably 0.01 to 50mg, most preferably 0.5 to 20mg of the active ingredient per unit dose (expressed as the weight of free base) which could be administered, for example, 1 to 4 times per day. The dose will depend on the route of administration and the condition being treated. It will be appreciated that it may be necessary to make routine variations to the dosage depending on the age and weight of the patient as well as the severity of the condition to be treated.

According to another aspect of the invention, compounds of general formula (I), and physiologically acceptable salts or solvates thereof, may be prepared by the general methods outlined hereinafter.

In the following description, the groups $R^1$ to $R^{11}$, Q and Im are as defined for compounds of general formula (I) unless otherwise stated.

According to a first general process (A), a compound of general formula (I) may be prepared by reacting a compound of general formula (II):

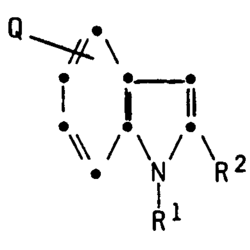

(II)

or a protected derivative thereof, with an acylating derivative of an acid of general formula (III) :

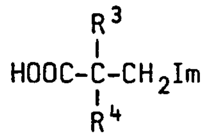

(III)

or a salt or protected derivative thereof, followed where necessary by removal of any protecting groups.

Suitable acylating derivatives of the acid (III) include acid halides (e.g. acid chlorides), anhydrides (e.g. symmetrical anhydrides or mixed anhydrides formed for example with pivaloyl chloride), amides, and nitriles.

Thus for example indoles of formula (II) may be acylated according to the Vilsmeier-Haack reaction, using a tertiary amide derivative of an acid of formula (III), such as the corresponding N,N-dimethylpropanamide compound, or a salt thereof, in the presence of a phosphoryl halide such as phosphorous oxychloride. This reaction may be effected in the presence or absence of a solvent. Solvents which may conveniently be employed include halogenated hydrocarbons such as 1,2-dichloroethane. The reaction temperature may be in the range 20 to 100 °C.

According to another general process (B), a compound of general formula (I) wherein $R^1$ represents the group $-CO_2R^8$, $-COR^8$, $-COR^8R^9$ or $-SO_2R^8$ may be prepared by acylating or sulphonylating as appropriate, a compound of formula (IV):

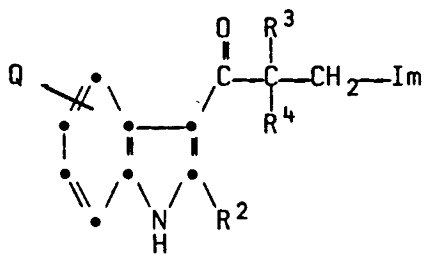

(IV)

or a protected derivative thereof, followed where necessary by removal of any protecting groups. The acylation/sulphonylation reaction may be effected using an appropriate acylating/sulphonylating agent according to conventional procedures.

Suitable acylating agents include acyl halides (e.g. an acyl chloride, bromide or iodide), mixed and symmetrical anhydrides (e.g. a symmetrical anhydride of formula $(R^8CO)_2O$), lower alkyl haloformates (e.g. lower alkyl chloroformates), carbamoyl halides (e.g. carbamoyl chlorides of formula $R^8R^9NCOCl$), carbonates and isocyanates (e.g. isocyanates of formula $R^8NCO$). Suitable sulphonylating agents include sulphonyl halides (e.g. an alkylsulphonyl or arylsulphonyl chloride, bromide or iodide) and sulphonates (e.g. hydrocarbylsulphonates such as p-toluenesulphonate).

The reaction may conveniently be effected in the presence of a base such as an alkali metal hydride (e.g. sodium or potassium hydride), an alkali metal carbonate (e.g. sodium or potassium carbonate), an alkali metal alkoxide (e.g. potassium t-butoxide), butyllithium, lithium diisopropylamide or an organic tertiary amine (e.g. triethylamine or pyridine).

Suitable solvents which may be employed in the acylation/sulphonylation of general process (B) include substituted amides (e.g. dimethylformamide or dimethylacetamide), ethers (e.g. tetrahydrofuran or dioxan), halogenated hydrocarbons (e.g. methylene chloride), nitriles (e.g. acetonitrile) and esters (e.g. ethyl acetate). The reaction may conveniently be effected at a temperature of from -10 to +150 °C.

According to another general process (C), a compound of general formula (I) may be converted into another compound of formula (I) using conventional techniques. Such conventional techniques include hydrogenation, alkylation, acylation and acid-catalysed cleavage using protection and deprotection where necessary.

Thus, according to one embodiment of the interconversion process (C), hydrogenation may be used to convert an alkenyl or an alkynyl substituent into an alkyl substituent or an alkynyl into an alkenyl substituent, or a benzyloxy substituent into a hydroxyl group.

Hydrogenation according to general process (C) may be effected using conventional procedures, for example using hydrogen in the presence of a noble metal catalyst (e.g. palladium, Raney nickel, platinum

or rhodium). The catalyst may be supported on, for example, charcoal, alumina or alternatively a homogeneous catalyst such as tris(triphenylphosphine)rhodium chloride may be used. The hydrogenation will generally be effected in a solvent such as an alcohol (e.g. methanol or ethanol), an ether (e.g. dioxan), a halogenated hydrocarbon (e.g. dichloromethane) or an ester (e.g. ethyl acetate) or mixtures thereof, and at a temperature in the range -20 to +100°C, preferably 0 to 50°C.

According to another embodiment of the interconversion process (C), a compound of formula (I) in which $R^1$ represents a $C_{1-6}$ alkyl, $C_{3-7}$ cycloalkyl, $C_{3-6}$ alkenyl, $C_{3-10}$ alkynyl, $C_{3-7}$ cycloalkyl$C_{1-4}$-alkyl or phenyl$C_{1-3}$alkyl group, or a compound in which at least one of $R^3$ and $R^4$ represents a $C_{1-6}$ alkyl group, or a compound in which at least one of $R^5$ and $R^6$ represents a $C_{1-6}$ alkyl, $C_{3-7}$ cycloalkyl, $C_{3-6}$ alkenyl or phenyl $C_{1-3}$ alkyl group, or a compound in which Q represents a $C_{1-4}$alkoxy or a phenyl$C_{1-3}$alkoxy group, or a compound in which $R^{10}$ and/or $R^{11}$ represents a $C_{1-4}$ alkyl or $C_{3-4}$ alkenyl group may be prepared by alkylating a compound of formula (I) where one or more of $R^1$, $R^3$, $R^4$, $R^5$, $R^6$, $R^{10}$ and $R^{11}$ represent a hydrogen atom, or Q represents a hydroxyl group.

The term 'alkylation' according to general process (C) thus also includes the introduction of other groups such as cycloalkyl, alkenyl or phenalkyl groups.

The above alkylation reactions may be effected using the appropriate alkylating agent selected from compounds of formula $R^{12}Z$ where $R^{12}$ represents a $C_{1-6}$ alkyl, $C_{3-7}$ cycloalkyl, $C_{3-6}$ alkenyl, $C_{3-10}$ alkynyl, $C_{3-7}$ cycloalkyl$C_{1-4}$ alkyl, or phenyl$C_{1-3}$alkyl group, and Z represents a leaving atom or group such as a halogen atom (e.g. chlorine, bromine or iodine), an acyloxy group (e.g. trifluoroacetyloxy or acetoxy), or a sulphonyloxy group (e.g. trifluoromethanesulphonyloxy, p-toluenesulphonyloxy or methanesulphonyloxy); or a sulphate of formula $(R^{12})_2SO_4$.

The alkylation reaction is conveniently carried out in an inert organic solvent such as a substituted amide (e.g. dimethylformamide), an ether (e.g. tetrahydrofuran) or an aromatic hydrocarbon (e.g. toluene), preferably in the presence of a base. Suitable bases included, for example, alkali metal hydrides (e.g. sodium hydride), alkali metal amides (e.g. sodium amide or lithium diisopropylamide), alkali metal carbonates (e.g. sodium carbonate) or an alkali metal alkoxide (e.g. sodium or potassium methoxide, ethoxide or t-butoxide). The reaction may conveniently be effected at a temperature in the range -80 to +100°C, preferably -80 to +50°C.

According to another embodiment of general process (C), a compound of formula (I) wherein $R^1$ represents $-CO_2R^8$, $-COR^8$, $-CONR^8R^9$ or $-SO_2R^8$ and Q is other than a hydrogen atom, may be prepared by acylating or sulphonylating a compound of formula (I) wherein $R^1$ represents a hydrogen atom. The acylation reactions may be effected using an appropriate acylating or sulphonylating agent according to conventional procedures as described, for example, in general process (B).

According to a yet further embodiment of general process (C), a compound of formula (I) in which Q represents a hydroxyl group may be prepared from the corresponding compound in which Q represents an alkoxy or benzyloxy group by acid-catalysed cleavage. The reaction may be effected using a Lewis acid such as boron tribromide or aluminium trichloride, in a solvent such as a halogenated hydrocarbon (e.g. dichloromethane). The reaction temperature may conveniently be in the range -80 to +100°C.

It should be appreciated that in the above transformations it may be necessary or desirable to protect any sensitive groups in the molecule of the compound in question to avoid undesirable side reactions. For example, it may be necessary to protect the keto group, for example, as a ketal or a thioketal. It may also be necessary to protect the indole and/or imidazole nitrogen atoms, for example with an arylmethyl (e.g. benzyl or trityl), alkyl (e.g. t-butyl), alkoxymethyl (e.g. methoxymethyl), acyl (e.g. benzyloxycarbonyl) or a sulphonyl (e.g. N,N-dimethylaminosulphonyl or p-toluenesulphonyl) group. When Q represents a hydroxyl group it may be necessary to protect the hydroxyl group, for example with an arylmethyl (e.g. benzyl or trityl) group.

Thus according to another general process (D), a compound of general formula (I) may be prepared by the removal of any protecting groups from a protected form of a compound of formula (I). Deprotection may be effected using conventional techniques such as those described in 'Protective Groups in Organic Synthesis' by Theodora W. Greene (John Wiley and Sons, 1981).

For example a ketal such as an alkyleneketal group may be removed by treatment with a mineral acid such as hydrochloric acid. A thioketal group may be cleaved by treatment with a mercuric salt, (e.g. mercuric chloride), in a suitable solvent, such as ethanol. An arylmethyl N-protecting group may be cleaved by hydrogenolysis in the presence of a catalyst (e.g. palladium on charcoal) and a trityl group may also be cleaved by acid hydrolysis (e.g. using dilute hydrochloric or acetic acid). An alkoxyalkyl group may be removed using a Lewis acid such as boron tribromide. An acyl group may be removed by hydrolysis under acidic or basic conditions (e.g. using hydrogen bromide or sodium hydroxide). A sulphonyl group may be removed by alkaline hydrolysis. An arylmethyl OH-protecting group may be cleaved under acidic conditions

(e.g. with dilute acetic acid, hydrobromic acid or boron tribromide) or by hydrogenolysis in the presence of a catalyst (e.g. palladium on charcoal).

Compounds of general formula (II) are either known or may be prepared from known compounds by conventional procedures.

Acylating derivatives of the acids of formula (III), as well as the acids (III), and compounds of general formula (IV) in which Q represents a hydrogen atom may be prepared, for example, by the methods described in published European Patent Specification No. 242973.

Where it is desired to isolate a compound of the invention as a salt, for example a physiologically acceptable salt, this may be achieved by reacting the compound of formula (I) in the form of the free base with an appropriate acid, preferably with an equivalent amount, in a suitable solvent such as an alcohol (e.g. ethanol or methanol), an aqueous alcohol (e.g. aqeous ethanol), an ester (e.g. ethyl acetate) or an ether (e.g. tetrahydrofuran).

Physiologically acceptable salts may also be prepared from other salts, including other physiologically acceptable salts, of the compound of formula (I) using conventional methods.

Individual enantiomers of the compounds of the invention may be obtained by resolution of a mixture of enantiomers (e.g. a racemic mixture) using conventional means, such as an optically active resolving acid; see for example 'Stereochemistry of Carbon Compounds' by E.L.Eliel (McGraw Hill 1962) and 'Tables of Resolving Agents' by S. H. Wilen.

The methods indicated above for preparing the compounds of the invention can be used as the last main step in the preparative sequence. The same general methods can be used for the introduction of the desired groups at an intermediate stage in the stepwise formation of the required compound, and it will be appreciated that these general methods can be combined in different ways in such multi-stage processes. The sequence of the reactions in multi-stage processes should of course be chosen so that the reaction conditions used do not affect groups in the molecule which are desired in the final product.

The invention is further illustrated by the following Examples. All temperatures are in °C. Thin layer chromatography (t.l.c.) was carried out over silica, and flash column chromatography (FCC) on silica (Merck 9385). Solvent System A as used for chromatography denotes dichloromethane:ethanol:0.88 ammonia solution. Organic extracts were dried over sodium sulphtae or magnesium sulphate. The following abbreviations are used: DMF-dimethylformamide, THF-tetrahydrofuran.

## Example 1

**3-(5-Methyl-1H-imidazol-4-yl)-1-(1,7-dimethyl-1H-indol-3-yl)propanone maleate**

A solution of N,N-dimethyl-3-(5-methyl-1H-imidazol-4-yl)-1-propanamide (300mg) in ethanol (15mℓ) was acidified (to pH 1) by the addition of ethanolic hydrogen chloride, and evaporated to yield an oil which was triturated with ether to give N,N- dimethyl-3-(5-methyl-1H-imidazol-4-yl)-1-propanamide dihydrochloride (270mg) as a powder. This was added to a mixture of 1,7-dimethylindole (195mg) and phosphorus oxychloride (0.15mℓ) and the mixture was heated at 85° in a closed vessel for 1.5h. The mixture was cooled, cold water (50mℓ) was added and the suspension was stirred for 0.5h. It was then washed with ethyl acetate (2x40mℓ; discarded), basified with 2N sodium carbonate and extracted with ethyl acetate (4x80mℓ). The combined, dried organic extracts were evaporated to give the free base of the title compound (232mg) as a solid which was dissolved in hot ethanol (15mℓ), and maleic acid (105mg) in warm ethanol (5mℓ) was added. The solution was evaporated and the residual solid was crystallised from ethanol (15mℓ) to give the title compound (235mg), m.p. 164-165°.

Analysis Found: C,63.4; H,5.9; N,10.5.

$C_{17}H_{19}N_3O.C_4H_4O_4$ requires C,63.5; H,5.8; N,10.6%.

Examples 2,3,4 and 5 were prepared in a similar manner to Example 1.

## Example 2

**3-(5-Methyl-1H-imidazol-4-yl)-1-(1,6-dimethyl-1H-indol-3-yl)propanone maleate**

N,N-Dimethyl-3-(5-methyl-1H-imidazol-4-yl)-1- propanamide (255mg) was converted to its dihydrochloride and reacted with 1,6-dimethyl-1H-indole (250mg) and phosphorus oxychloride (0.19mℓ) at 85° for 2.5h. Work-up and salt formation gave the title compound (247mg), m.p. 167-168°.

Analysis Found: C,63.3; H,5.9; N,10.4.

$C_{17}H_{19}N_3O.C_4H_4O_4$ requires C,63.5; H,5.8; N,10.6%.

Example 3

**3-(5-Methyl-1H-imidazol-4-yl)-1-(5-fluoro-1-methyl-1H-indol-3-yl)propanone maleate**

The reaction of N,N-dimethyl-3-(5-methyl-1H-imidazol-4-yl)-1-propanamide dihydrochloride (505mg), 5-fluoro-1-methyl-1H-indole (350mg) and phosphorus oxychloride (0.23mℓ) followed by work-up and salt formation gave the title compound (483mg), m.p. 172-173°.
Analysis Found: C,59.9; H,5.1; N,10.3.
$C_{16}H_{16}FN_3O.C_4H_4O_4$ requires C,59.9; H,5.0; N,10.5%.

Example 4

**3-(5-Methyl-1H-imidazol-4-yl)-1-(1,5-dimethyl-1H-indol-3-yl)propanone maleate**

The reaction of N,N-dimethyl-3-(5-methyl-1H-imidazol-4-yl)-1-propanamide dihydrochloride (317mg), 1,5-dimethyl-1H-indole (220mg) and phosphorus oxychloride (0.17mℓ) followed by work-up and salt formation gave the title compound (327mg), m.p. 168-169°.
Analysis Found: C,63.2; H,5.8; N,10.7.
$C_{17}H_{19}N_3O.C_4H_4O_4$ requires C,63.5; H,5.8; N,10.6%.

Example 5

**3-(1H-Imidazol-4-yl)-1-(5-fluoro-1-methyl-1H-indol-3-yl)propanone maleate**

The reaction of N,N-dimethyl-3-(1H-imidazol-4-yl)-1-propanamide hydrochloride (220mg), and 5-fluoro-1-methyl-1H-indole (146mg) and phosphorus oxychloride (0.10mℓ) followed by work-up and salt formation gave the title compound (200mg). Further recrystallisation from ethanol (8ml) gave the title compound -(120mg), m.p. 153-154°.
Analysis Found : C,58.7; H,4.7; N,10.6.
$C_{15}H_{14}FN_3O.C_4H_4O_4$ requires C,58.9; H,4.7; N,10.4%.

Example 6

**1-(1-Methoxycarbonyl-1H-indol-3-yl)-3-(5-methyl-1H-imidazol-4-yl)-1-propanone maleate**

A suspension of 3-[5-methyl-1-(triphenylmethyl)-1H-imidazol-4-yl]-1-(1H-indol-3-yl)-1-propanone (500mg) in dry DMF (3mℓ) was added dropwise to a stirred suspension of sodium hydride (73% dispersion in oil; 40mg) in dry DMF (1mℓ) under nitrogen. After 15 min. methyl chloroformate (0.093mℓ) was added and the mixture was stirred for 4.5h. Water (50mℓ) was added and the suspension was extracted with dichloromethane (3x25mℓ). The combined, dried organic extracts were evaporated to give an oil (ca. 710mg) which was purified by FCC (column made up in ethyl acetate:hexane:triethylamine 80:19:1) eluting with ethyl acetate:hexane (4:1) to give a foam (320mg). This was dissolved in a mixture of dry THF (9mℓ), acetic acid (9mℓ) and water (9mℓ) and heated at reflux for 1h. The mixture was poured into saturated potassium carbonate solution (60mℓ) and extracted with dichloromethane (3x30mℓ). The combined, dried organic extracts were dried to give a solid (288mg) which was purified by FCC eluting with System A (200:10:1) to give a solid (174mg). This was dissolved in ethanol (2mℓ) and treated with a solution of maleic acid (68mg) in ethanol (0.5mℓ). The solvent was removed in vacuo and the residue was triturated with dry ether (3x5mℓ) to give the title compound (180mg), m.p. 167-169°.
Water Assay Found 1.38% w/w ≡ 0.33mol $H_2O$.
Analysis Found C,58.0; H,4.9; N,9.5.
$C_{17}H_{17}N_3O_3.C_4H_4O_4.0.33H_2O$ requires C,58.2; H,5.0; N,9.7%.

Example 7

**N,N-Dimethyl-3-[3-(5-methyl-1H-imidazol-4-yl]-1-oxopropyl]-1H-indole-1-carboxamide maleate**

**(i) N,N-Dimethyl-3-[3-[5-methyl-1-(triphenylmethyl)-1H-imidazol-4-yl]-1-oxopropyl]-1H-indole-1-carboxamide**

A solution of 3-[5-methyl-1-(triphenylmethyl)-1H-imidazol-4-yl]-1-(1H-indol-3-yl)-1-propanone (500mg) in dry DMF (3mℓ) was added dropwise to a stirred suspension of sodium hydride (73% dispersion in oil; 40mg) in dry DMF (1mℓ) under nitrogen. After 15 min. dimethylcarbamyl chloride (0.11mℓ) was added and the mixture was stirred for 20h. Water (50mℓ) was added and the suspension was extracted with dichloromethane (3 x 25mℓ). The combined, dried organic extracts were evaporated to give an oil (815mg) which was purified by FCC (column made up in ethyl acetate:triethylamine 99:1) eluting with ethyl acetate to give the title compound (445mg) as a foam, t.l.c. on triethylamine impregnated silica (ethyl acetate) Rf 0.20.

**(ii) N,N-Dimethyl-3-[3-(5-methyl-1H-imidazol-4-yl]-1-oxopropyl]-1H-indole-1-carboxamide maleate**

A solution of N,N-dimethyl-3-[3-[5-methyl-1-(triphenylmethyl)-1H-imidazol-4-yl]-1-oxopropyl]-1H-indole-1-carboxamide (400mg) in a mixture of acetic acid (12mℓ), water (12mℓ) and THF (12mℓ) was heated at reflux for 3h. The mixture was poured into saturated potassium carbonate solution (80mℓ) and extracted with dichloromethane (3 x 40mℓ). The combined, dried organic extracts were evaporated to give a gum (420mg) which was purified by FCC eluting with System A (200:10:1) to give an oil (200mg). This was dissolved in ethanol (ca. 3mℓ) and treated with a solution of maleic acid (75mg) in ethanol (ca. 1mℓ). The solvent was removed in vacuo and the residue was triturated with dry ether (5 x 5mℓ) to give the title compound (218mg), m.p. 129-130°.
Analysis Found: C,59.9; H,5.7; N,12.4.
$C_{18}H_{20}N_4O_2.C_4H_4O_4$ requires C,60.0; H,5.5; N,12.7%.

The following examples illustrate pharmaceutical formulations according to the invention. The term "active ingredient" is used herein to represent a compound of formula (I).

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression Tablet

| | mg/tablet |
|---|---|
| Active Ingredient | 0.50 |
| Calcium Hydrogen Phosphate BP* | 87.25 |
| Croscarmellose Sodium NF | 1.80 |
| Magnesium Stearate BP | 0.45 |
| Compression weight | 90.00 |

* of a grade suitable for direct compression.

The active ingredient is passed through a 60 mesh sieve, blended with the calcium hydrogen phosphate, croscarmellose sodium and magnesium stearate. The resultant mix is compressed into tablets using a Manesty F3 tablet machine fitted with 5.5mm, flat bevelled edge punches.

Tablets of other strengths may be prepared by altering the ratio of active ingredient to excipients or the compression weight and using punches to suit.

CAPSULES

| | mg/capsule |
|---|---|
| Active Ingredient | 0.5 |
| * Starch 1500 | 98.5 |
| Magnesium Stearate BP | 1.0 |
| Fill Weight | 100.0 |

* a form of directly compressible starch.

The active ingredient is sieved and blended with the excipients. The mix is filled into size No. 2 hard gelatin capsules using suitable machinery. Other doses may be prepared by altering the fill weight and if necessary changing the capsule size to suit.

SYRUP

This may be either a sucrose or sucrose free presentation.

| Sucrose-Free Syrup | | mg/5ml dose |
|---|---|---|
| Active Ingredient | | 0.5 |
| Hydroxypropylmethylcellulose USP (viscosity type 4000) | | 22.5 |
| Buffer ) Flavour ) Colour ) Preservative ) Sweetener ) | | as required |
| Purified Water BP | to | 5.0ml |

The hydroxypropylmethylcellulose is dispersed in hot water, cooled and then mixed with an aqueous solution containing the active ingredient and the other components of the formulation. The resultant solution is adjusted to volume and mixed. The syrup is clarified by filtration.

INJECTION FOR INTRAVENOUS ADMINISTRATION

| | mg/mℓ | |
|---|---|---|
| Active ingredient | 0.05 | 0.5 |
| Sodium Chloride BP | as required | as required |
| Water for Injection BP to | 1.0mℓ | 1.0mℓ |

Sodium chloride may be added to adjust the tonicity of the solution and the pH may be adjusted, using acid or alkali, to that of optimum stability and/or facilitate solution of the active ingredient. Alternatively, suitable buffer salts may be used.

The solution is prepared, clarified and filled into appropriate size ampoules sealed by fusion of the glass. The injection is sterilised by heating in an autoclave using one of the acceptable cycles. Alternatively,

the solution may be sterilised by filtration and filled into sterile ampoules under aseptic conditions. The solution may be packed under an inert atmosphere of nitrogen or other suitable gas.

**Claims**

**Claims for the following Contracting States : AT, BE, CH, DE, FR, GB, IT, LI, LU, NL, SE**

1. Compounds of the general formula (I):

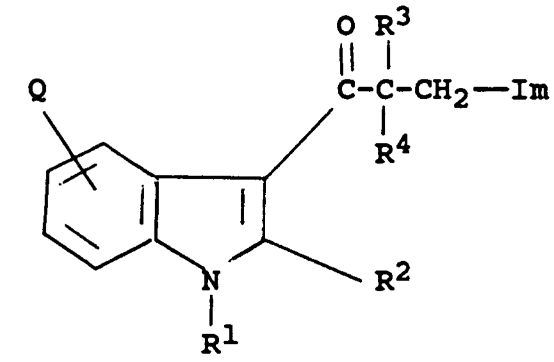

(I)

wherein Im represents an imidazolyl group of formula :

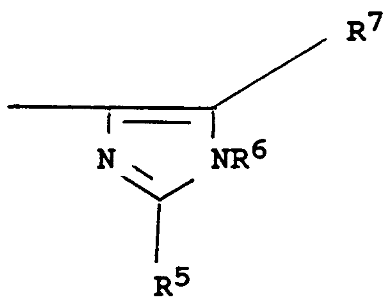

or

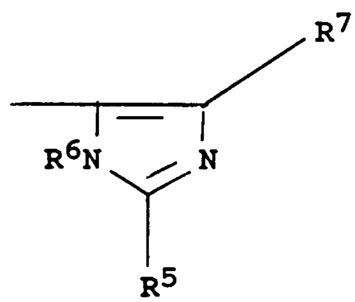

$R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl, phenyl$C_{1-3}$alkyl, $-CO_2R^8$,$-COR^8$, $-CONR^8R^9$ or $-SO_2R^8$ -(wherein $R^8$ and $R^9$, which may be the same or different, each represents a hydrogen atom, a $C_{1-6}$alkyl or $C_{3-7}$cycloalkyl group, or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^8$ does not represent a hydrogen atom when $R^1$ represents a group $-CO_2R^8$ or $-SO_2R^8$);
$R_2$ represents a hydrogen atom or a $C_{1-6}$alkyl, $C_{3-6}$alkenyl, $C_{3-7}$cycloalkyl, phenyl or phenyl$C_{1-3}$ alkyl group;
$R^3$ and $R^4$, which may be the same or different, each represents a hydrogen atom or a $C_{1-6}$alkyl group;
one of the gruops represented by $R^5$, $R^6$ and $R^7$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;
Q represents a hydrogen atom or a halogen atom or a hydroxy, $C_{1-4}$alkoxy, phenyl$C_{1-3}$alkoxy or $C_{1-6}$alkyl group or a group $-NR^{10}R^{11}$ or $-CONR^{10}R^{11}$ (wherein $R^{10}$ and $R^{11}$, which may be the same or different, each represents a hydrogen atom or a $C_{1-4}$alkyl or $C_{3-4}$alkenyl group, or together with the nitrogen atom to which they are attached form a saturated 5 to 7 membered ring);
and with the proviso that when Q represents a hydrogen atom $R^1$ represents $-CO_2R^8$, $-COR^8$, $-CONR^8R^9$ or $-SO_2R^8$;
and physiologically acceptable salts and solvates thereof.

2. Compounds as claimed in claim 1 in which $R^1$ represents a hydrogen atom or a $C_{1-3}$alkyl, $C_{3-4}$alkenyl, $C_{3-4}$alkynyl, $C_{5-6}$cycloalkyl, benzyl, N,N-di$C_{1-3}$alkylcarboxamido or $C_{1-3}$alkoxycarbonyl group.

3. Compounds as claimed in claim 1 or 2 in which $R^2$ represents a hydrogen atom or a $C_{1-3}$alkyl group.

4. Compounds as claimed in any of claims 1 to 3 in which $R^3$ and $R^4$ each independently represent a hydrogen atom or a $C_{1-3}$alkyl group.

5. Compounds as claimed in any of claims 1 to 4 in which $R^5$, $R^6$ and $R^7$ each represent a hydrogen atom or a $C_{1-3}$ alkyl group.

6. Compounds as claimed in any of claims 1 to 4 in which $R^5$ and $R^6$ both represent hydrogen atoms and $R^7$ represents a $C_{1-6}$alkyl, $C_{3-7}$cycloalkyl, $C_{3-6}$alkenyl, or phenyl$C_{1-3}$alkyl group.

7. Compounds as claimed in any of claims 1 to 6 in which Q represents a hydrogen atom, a halogen atom or a hydroxy, $C_{1-3}$alkoxy or $C_{1-3}$alkyl group.

8. Compounds as claimed in claim 1 in which $R^1$ represents a $C_{1-3}$alkyl, N,N-di$C_{1-3}$alkylcarboxamido or $C_{1-3}$alkoxycarbonyl group; $R^2$ represents a hydrogen atom; $R^3$ and $R^4$ both represent hydrogen atoms; $R^5$ and $R^6$ each represent a hydrogen atom; $R^7$ represents a hydrogen atom or a $C_{1-3}$alkyl group; and Q represents a hydrogen or a halogen atom or a $C_{1-3}$alkyl group.

9. A compound as claimed in claim 1 which is 3-(5-methyl-1H-imidazol-4-yl)-1-(1,7-dimethyl-1H-indol-3-yl)propanone or a physiologically acceptable salt or solvate thereof.

10. A process for the preparation of a compound of general formula (I) as defined in any of claims 1 to 9 or a physiologically acceptable salt or solvate thereof, which comprises:
(A) reacting a compound of general formula (II)

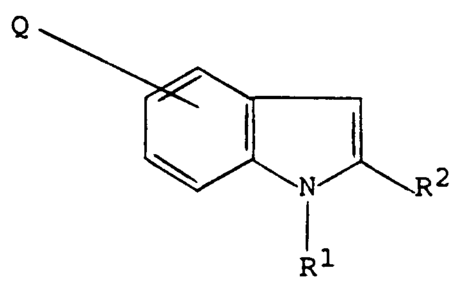

(II)

or a protected derivative thereof with an acylating derivative of an acid of general formula (III)

$$HOOC-C(R^3)(R^4)-CH_2Im \quad \text{(III)}$$

or a salt or protected derivative thereof, followed if necessary by removal of any protecting groups present; or
(B) for the preparation of a compound of formula (I) in which $R^1$ represents the group $-CO_2R^8$, $-COR^8$, $-COR^8R^9$ or $-SO_2R^8$, acylating or sulphonylating as appropriate a compound of formula (IV)

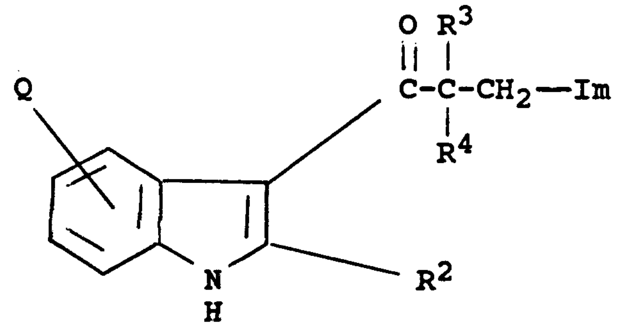

(IV)

or a protected derivative thereof, followed if necessary by removal or any protecting groups present; or

(C) converting a compound of formula (I) or a salt or protected derivative thereof into another compound of formula (I) using conventional techniques; or

(D) removing protecting group(s) from a protected form of a compound of formula (I);

and, when the compound of formula (I) is obtained as a mixture of enantiomers, optionally resolving the mixture to obtain the desired enantiomer;

and/or where the compound of formula (I) is in the form of a free base, optionally converting the free base into a salt.

**11.** A pharmaceutical composition comprising at least one compound of general formula (I) as defined in any of claims 1 to 9 or a physiologically acceptable salt or solvate thereof together with at least one physiologically acceptable carrier or excipient.

**12.** A compound of general formula (I) as defined in claim 1 for use as an active therapeutic agent.

**Claims for the following Contracting States : ES, GR**

**1.** A process for the preparation of compounds of the general formula (I):

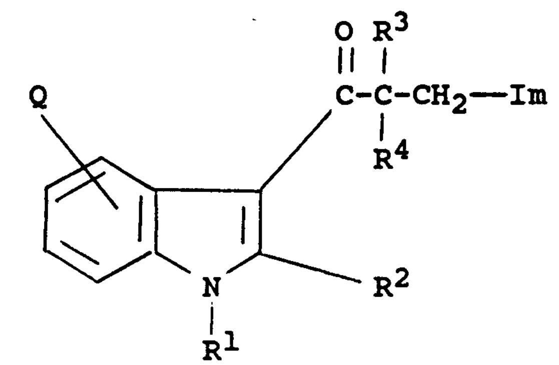

(I)

wherein Im represents an imidazolyl group of formula :

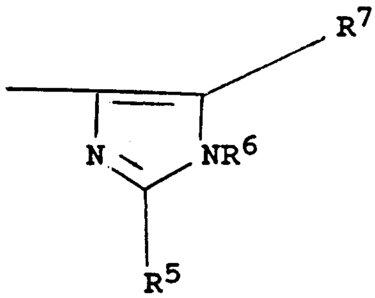

or

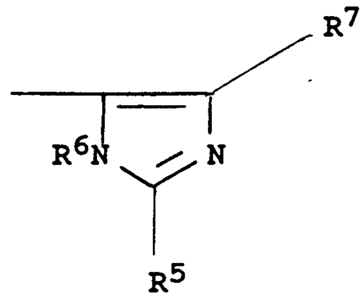

$R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl, phenyl$C_{1-3}$alkyl, $-CO_2R^8$,$-COR^8$, $-CONR^8R^9$ or $-SO_2R^8$ - (wherein $R^8$ and $R^9$, which may be the same or different, each represents a hydrogen atom, a $C_{1-6}$alkyl or $C_{3-7}$cycloalkyl group, or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^8$ does not represent a hydrogen atom when $R^1$ represents a group $-CO_2R^8$ or $-SO_2R^8$);

$R_2$ represents a hydrogen atom or a $C_{1-6}$alkyl, $C_{3-6}$alkenyl, $C_{3-7}$cycloalkyl, phenyl or phenyl$C_{1-3}$ alkyl group;

$R^3$ and $R^4$, which may be the same or different, each represents a hydrogen atom or a $C_{1-6}$alkyl group;

one of the groups respresented by $R^5$, $R^6$ and $R^7$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;

Q represents a hydrogen atom or a halogen atom or a hydroxy, $C_{1-4}$alkoxy, phenyl$C_{1-3}$alkoxy or $C_{1-6}$alkyl group or a group $-NR^{10}R^{11}$ or $-CONR^{10}R^{11}$ (wherein $R^{10}$ and $R^{11}$, which may be the same or different, each represents a hydrogen atom or a $C_{1-4}$alkyl or $C_{3-4}$alkenyl group, or together with the nitrogen atom to which they are attached form a saturated 5 to 7 membered ring);

and with the proviso that when Q represents a hydrogen atom $R^1$ represents $-CO_2R^8$, $-COR^8$, $-CONR^8R^9$ or $-SO_2R^8$;

and physiologically acceptable salts and solvates thereof, which comprises:

(A) reacting a compound of general formula (II)

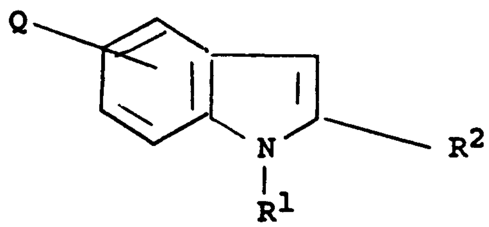

(II)

or a protected derivative thereof with an acylating derivative of an acid of general formula (III)

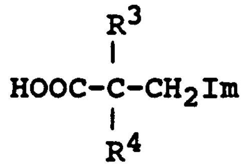

(III)

or a salt or protected derivative thereof, followed if necessary by removal of any protecting groups present; or

(B) for the preparation of a compound of formula (I) in which $R^1$ represents the group $-CO_2R^8$, $-COR^8$, $-COR^8R^9$ or $-SO_2R^8$, acylating or sulphonylating as appropriate a compound of formula (IV)

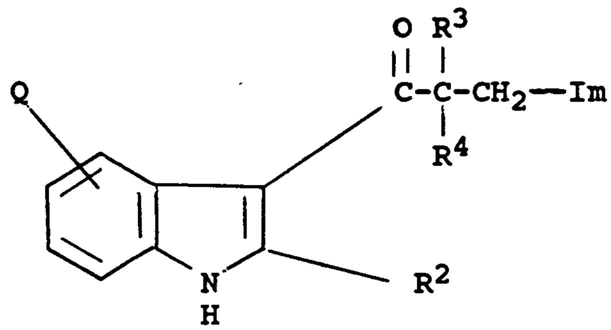

(IV)

or a protected derivative thereof, followed if necessary by removal or any protecting groups present; or

(C) converting a compound of formula (I) or a salt or protected derivative thereof into another compound of formula (I) using conventional techniques; or

(D) removing protecting group(s) from a protected form of a compound of formula (I);

and, when the compound of formula (I) is obtained as a mixture of enantiomers, optionally resolving the mixture to obtain the desired enantiomer;

and/or where the compound of formula (I) is in the form of a free base, optionally converting the free base into a salt.

**2.** A process as claimed in claim 1 for the production of compounds in which $R^1$ represents a hydrogen atom or a $C_{1-3}$alkyl, $C_{3-4}$alkenyl, $C_{3-4}$alkynyl, $C_{5-6}$cycloalkyl, benzyl, N,N-di$C_{1-3}$alkylcarboxamido or $C_{1-3}$alkoxycarbonyl group.

**3.** A process as claimed in claim 1 or 2 for the production of compounds in which $R^2$ represents a hydrogen atom or a $C_{1-3}$alkyl group.

**4.** A process as claimed in any of claims 1 to 3 for the production of compounds in which $R^3$ and $R^4$ each independently represent a hydrogen atom or a $C_{1-3}$alkyl group.

**5.** A process as claimed in any of claims 1 to 4 for the production of compounds in which $R^5$, $R^6$ and $R^7$ each represent a hydrogen atom or a $C_{1-3}$ alkyl group.

**6.** A process as claimed in any of claims 1 to 4 for the production of compounds in which $R^5$ and $R^6$ both represent hydrogen atoms and $R^7$ represents a $C_{1-6}$alkyl, $C_{3-7}$cycloalkyl, $C_{3-6}$alkenyl, or phenyl$C_{1-3}$alkyl group.

**7.** A process as claimed in any of claims 1 to 6 for the production of compounds in which Q represents a hydrogen atom, a halogen atom or a hydroxy, $C_{1-3}$alkoxy or $C_{1-3}$alkyl group.

**8.** A process as claimed in claim 1 for the production of compounds in which $R^1$ represents a $C_{1-3}$alkyl, N,N-di$C_{1-3}$alkylcarboxamido or $C_{1-3}$alkoxycarbonyl group; $R^2$ represents a hydrogen atom; $R^3$ and $R^4$ both represent hydrogen atoms; $R^5$ and $R^6$ each represent a hydrogen atom; $R^7$ represents a hydrogen atom or a $C_{1-3}$alkyl group; and Q represents a hydrogen or a halogen atom or a $C_{1-3}$ alkyl group.

**9.** A process as claimed in claim 1 for the production of 3-(5-methyl-1H-imidazol-4-yl)-1-(1,7-dimethyl-1H-indol-3-yl)propanone and physiologically acceptable salts and solvates thereof.

**Revendications**

**Revendications pour les Etats contractants suivants : AT, BE, CH, DE, FR, GB, IT, LI, LU, NL, SE**

**1.** Composés de formule générale (I) :

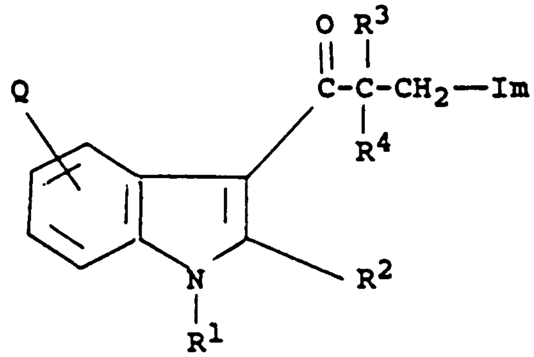

(I)

dans laquelle Im représente un groupe imidazolyle de formule :

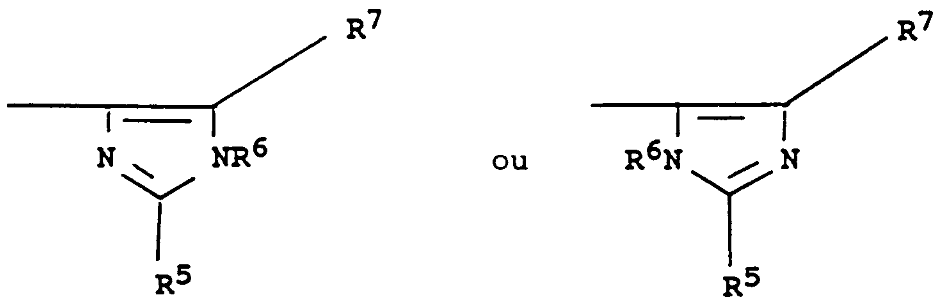

ou

$R^1$ représente un atome d'hydrogène ou un groupe choisi parmi les groupes alkyle en $C_1$-$C_6$, alcényle en $C_3$-$C_6$, alcynyle en $C_3$-$C_{10}$, cycloalkyle en $C_3$-$C_7$, cycloalkyl(en $C_3$-$C_7$)alkyle (en $C_1$-$C_4$), phényle, phényl(alkyle en $C_1$-$C_3$), $-CO_2R^8$, $-COR^8$, $-CONR^8R^9$ ou $-SO_2R^8$ (où $R^8$ et $R^9$, qui peuvent être identiques ou différents, représentent chacun un atome d'hydrogène, un groupe alkyle en $C_1$-$C_6$ ou cycloalkyle en $C_3$-$C_7$, ou un groupe phényle ou phényl(alkyle en $C_1$-$C_4$), dans lequel le groupe phényle est éventuellement substitué par un ou plusieurs groupes alkyle en $C_1$-$C_4$, alcoxy en $C_1$-$C_4$ ou hydroxy ou par des atomes d'halogène, à condition que $R^8$ ne représente pas un atome d'hydrogène lorsque $R^1$ représente un groupe $-CO_2R^8$ ou $-SO_2R^8$);
$R^2$ représente un atome d'hydrogène ou un groupe alkyle en $C_1$-$C_6$, alcényle en $C_3$-$C_6$, cycloalkyle en $C_3$-$C_7$, phényle ou phényl(alkyle en $C_1$-$C_3$);
$R^3$ et $R^4$, qui peuvent être identiques ou différents, représentent chacun un atome d'hydrogène ou un groupe alkyle en $C_1$-$C_6$;
l'un des groupes représentés par $R^5$, $R^6$ et $R^7$ est un atome d'hydrogène ou un groupe alkyle en $C_1$-$C_6$, cycloalkyle en $C_3$-$C_7$, alcényle en $C_3$-$C_6$, phényle ou phényl(alkyle en $C_1$-$C_3$), et chacun des deux autres groupes, qui peuvent être identiques ou différents, représentent un atome d'hydrogène ou un groupe alkyle en $C_1$-$C_6$;
Q représente un atome d'hydrogène ou un atome d'halogène ou un groupe hydroxy, alcoxy en $C_1$-$C_4$, phényl(alcoxy en $C_1$-$C_3$) ou alkyle en $C_1$-$C_6$, ou un groupe $-NR^{10}R^{11}$ ou $-CONR^{10}R^{11}$ (où $R^{10}$ et $R^{11}$, qui peuvent être identiques ou différents, représentent chacun un atome d'hydrogène ou un groupe alkyle en $C_1$-$C_4$ ou alcényle en $C_3$-$C_4$, ou bien forment, avec l'atome d'azote auquel ils sont fixés, un noyau saturé à 5-7 chaînons);
et à condition que, lorsque Q représente un atome d'hydrogène, $R^1$ représente $-CO_2R^8$, $-COR^8$, $-CONR^8R^9$ ou $-SO_2R^8$;
et leurs sels et solvates physiologiquement acceptables.

**2.** Composés selon la revendication 1, dans lesquels $R^1$ représente un atome d'hydrogène ou un groupe alkyle en $C_1$-$C_3$, alcényle en $C_3$-$C_4$, alcynyle en $C_3$-$C_4$, cycloalkyle en $C_5$-$C_6$, benzyle, N,N-di(alkyl en $C_1$-$C_3$)carboxamido ou (alcoxy en $C_1$-$C_3$)carbonyle.

**3.** Composés selon la revendication 1 ou 2, dans lesquels $R^2$ représente un atome d'hydrogène ou un groupe alkyle en $C_1$-$C_3$.

**4.** Composés selon l'une quelconque des revendications 1 à 3, dans lesquels $R^3$ et $R^4$ représentent chacun, indépendamment, un atome d'hydrogène ou un groupe alkyle en $C_1$-$C_3$.

**5.** Composés selon l'une quelconque des revendications 1 à 4, dans lesquels $R^5$, $R^6$ et $R^7$ représentent chacun un atome d'hydrogène ou un groupe alkyle en $C_1$-$C_3$.

**6.** Composés selon l'une quelconque des revendications 1 à 4, dans lesquels $R^5$ et $R^6$ représentent tous deux des atomes d'hydrogène et $R^7$ représente un groupe alkyle en $C_1$-$C_6$, cycloalkyle en $C_3$-$C_7$, alcényle en $C_3$-$C_6$ ou phényl(alkyle en $C_1$-$C_3$).

**7.** Composés selon l'une quelconque des revendications 1 à 6, dans lesquels Q représente un atome d'hydrogène, un atome d'halogène ou un groupe hydroxy, alcoxy en $C_1$-$C_3$ ou alkyle en $C_1$-$C_3$.

**8.** Composés selon la revendication 1, dans lesquels $R^1$ représente un groupe alkyle en $C_1$-$C_3$, N,N-di-(alkyl en $C_1$-$C_3$)-carboxamido ou (alcoxy en $C_1$-$C_3$)carbonyle; $R^2$ représente un atome d'hydrogène; $R^3$ et $R^4$ représentent tous deux des atomes d'hydrogène; $R^5$ et $R^6$ représentent chacun un atome d'hydrogène; $R^7$ représente un atome d'hydrogène ou un groupe alkyle en $C_1$-$C_3$; et Q représente un atome d'hydrogène ou d'halogène, ou un groupe alkyle en $C_1$-$C_3$.

**9.** Composé selon la revendication 1, qui est la 3-(5-méthyl-1H-imidazole-4-yl)-1-(1,7-diméthyl-1H-indole-3-yl)-propanone ou un de ses sels et solvates physiologiquement acceptables.

**10.** Procédé de préparation d'un composé de formule générale (I) tel que défini dans l'une quelconque des revendications 1 à 9, ou d'un de ses sels ou solvates physiologiquement acceptables, qui comprend :

(A) la réaction d'un composé de formule générale (II) :

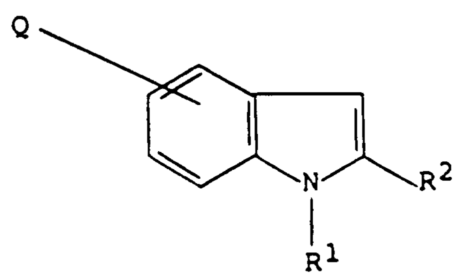

(II)

ou d'un de ses dérivés protégés, avec un dérivé d'acylation d'un acide de formule générale (III) :

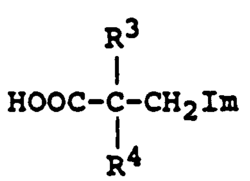

(III)

ou un de ses sels ou dérivés protégés, suivie, le cas échéant, de l'élimination des groupes protecteurs éventuellement présents; ou

(B) pour la préparation d'un composé de formule (I), dans laquelle $R^1$ représente le groupe $-CO_2R^8$, $-COR^8$, $-COR^8R^9$ ou $-SO_2R^8$, l'acylation ou la sulfonylation, selon les cas, d'un composé de formule (IV) :

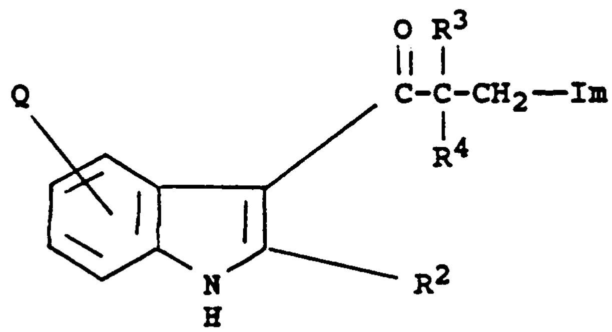

(IV)

ou d'un de ses dérivés protégés, suivie, le cas échéant, de l'élimination des groupes protecteurs éventuellement présents; ou

(C) la transformation d'un composé de formule (I) ou d'un de ses sels ou dérivés protégés en un autre composé de formule (I) par des techniques classiques; ou

(D) l'élimination du ou des groupes protecteurs de la forme protégée d'un composé de formule (I);

et, si le composé de formule (I) est obtenu sous forme d'un mélange d'énantiomères, la résolution éventuelle du mélange pour donner l'énantiomère désiré;

et/ou, si le composé de formule (I) est sous forme de base libre, la transformation éventuelle de la base libre en sel.

**11.** Composition pharmaceutique comprenant au moins un composé de formule générale (I) tel que défini dans l'une quelconque des revendications 1 à 9, ou un de ses sels ou solvates physiologiquement acceptables, avec au moins un véhicule ou excipient physiologiquement acceptable.

**12.** Composé de formule générale (I) tel que défini dans la revendication 1 à utiliser comme agent thérapeutique actif.

**Revendications pour les Etats contractants suivants : ES, GR**

**1.** Procédé de préparation de composés de formule générale (I) :

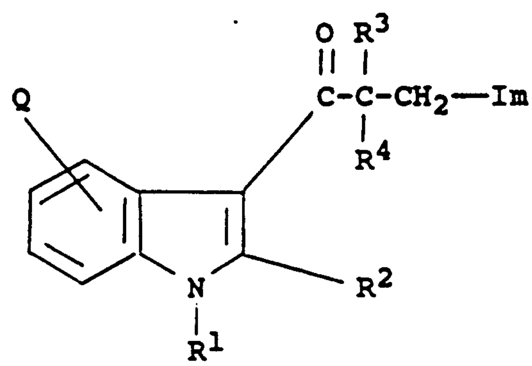

(I)

dans laquelle Im représente un groupe imidazolyle de formule :

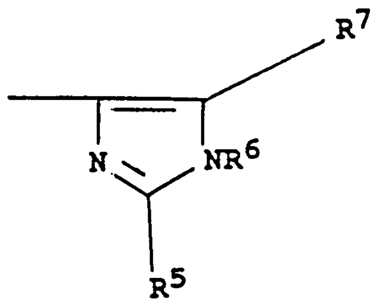 ou 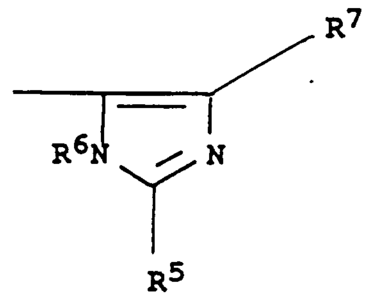

$R^1$ représente un atome d'hydrogène ou un groupe choisi parmi les groupes alkyle en $C_1$-$C_6$, alcényle en $C_3$-$C_6$, alcynyle en $C_3$-$C_{10}$, cycloalkyle en $C_3$-$C_7$, cycloalkyl(en $C_3$-$C_7$)alkyle (en $C_1$-$C_4$), phényle, phényl(alkyle en $C_1$-$C_3$), $-CO_2R^8$, $-COR^8$, $-CONR^8R^9$ ou $-SO_2R^8$ (où $R^8$ et $R^9$, qui peuvent être identiques ou différents, représentent chacun un atome d'hydrogène, un groupe alkyle en $C_1$-$C_6$ ou cycloalkyle en $C_3$-$C_7$, ou un groupe phényle ou phényl(alkyle en $C_1$-$C_4$), dans lequel le groupe phényle est éventuellement substitué par un ou plusieurs groupes alkyle en $C_1$-$C_4$, alcoxy en $C_1$-$C_4$ ou hydroxy ou par des atomes d'halogène, à condition que $R^8$ ne représente pas un atome d'hydrogène lorsque $R^1$ représente un groupe $-CO_2R^8$ ou $-SO_2R^8$);

$R^2$ représente un atome d'hydrogène ou un groupe alkyle en $C_1$-$C_6$, alcényle en $C_3$-$C_6$, cycloalkyle en $C_3$-$C_7$, phényle ou phényl(alkyle en $C_1$-$C_3$);

$R^3$ et $R^4$, qui peuvent être identiques ou différents, représentent chacun un atome d'hydrogène ou un groupe alkyle en $C_1$-$C_6$;

l'un des groupes représentés par $R^5$, $R^6$ et $R^7$ est un atome d'hydrogène ou un groupe alkyle en $C_1$-$C_6$, cycloalkyle en $C_3$-$C_7$, alcényle en $C_3$-$C_6$, phényle ou phényl(alkyle en $C_1$-$C_3$), et chacun des deux autres groupes, qui peuvent être identiques ou différents, représentent un atome d'hydrogène ou un groupe alkyle en $C_1$-$C_6$;

Q représente un atome d'hydrogène ou un atome d'halogène ou un groupe hydroxy, alcoxy en $C_1$-$C_4$, phényl(alcoxy en $C_1$-$C_3$) ou alkyle en $C_1$-$C_6$, ou un groupe $-NR^{10}R^{11}$ ou $-CONR^{10}R^{11}$ (où $R^{10}$ et $R^{11}$, qui peuvent être identiques ou différents, représentent chacun un atome d'hydrogène ou un groupe alkyle en $C_1$-$C_4$ ou alcényle en $C_3$-$C_4$, ou bien forment, avec l'atome d'azote auquel ils sont fixés, un noyau saturé à 5-7 chaînons);

et à condition que, lorsque Q représente un atome d'hydrogène, $R^1$ représente $-CO_2R^8$, $-COR^8$, $-CONR^8R^9$ ou $-SO_2R^8$;

et de leurs sels et solvates physiologiquement acceptables, qui comprend :

(A) la réaction d'un composé de formule générale (II) :

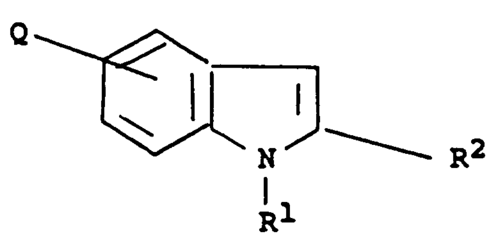 (II)

ou d'un de ses dérivés protégés, avec un dérivé d'acylation d'un acide de formule générale (III) :

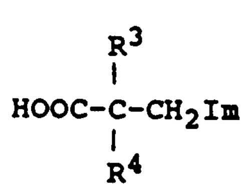

ou un de ses sels ou dérivés protégés, suivie, le cas échéant, de l'élimination des groupes protecteurs éventuellement présents; ou
(B) pour la préparation d'un composé de formule (I), dans laquelle $R^1$ représente le groupe $-CO_2R^8$, $-COR^8$, $-COR^8R^9$ ou $-SO_2R^8$, l'acylation ou la sulfonylation, selon les cas, d'un composé de formule (IV) :

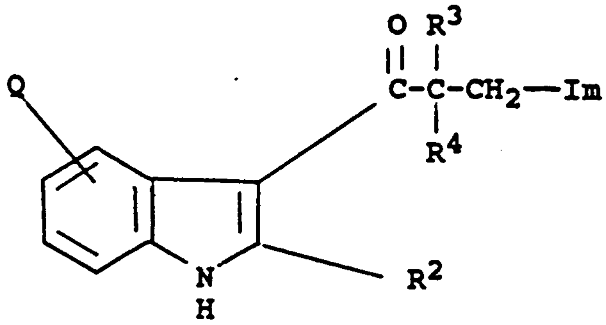

(IV)

ou d'un de ses dérivés protégés, suivie, le cas échéant, de l'élimination des groupes protecteurs éventuellement présents; ou
(C) la transformation d'un composé de formule (I) ou d'un de ses sels ou dérivés protégés en un autre composé de formule (I) par des techniques classiques; ou
(D) l'élimination du ou des groupes protecteurs de la forme protégée d'un composé de formule (I);
et, si le composé de formule (I) est obtenu sous forme d'un mélange d'énantiomères, la résolution éventuelle du mélange pour donner l'énantiomère désiré;
et/ou, si le composé de formule (I) est sous forme de base libre, la transformation éventuelle de la base libre en sel.

**2.** Procédé selon la revendication 1, pour la production de composés dans lesquels $R^1$ représente un atome d'hydrogène ou un groupe alkyle en $C_1$-$C_3$, alcényle en $C_3$-$C_4$, alcynyle en $C_3$-$C_4$, cycloalkyle en $C_5$-$C_6$, benzyle, N,N-di(alkyl en $C_1$-$C_3$)carboxamido ou (alcoxy en $C_1$-$C_3$)carbonyle.

**3.** Procédé selon la revendication 1 ou 2, pour la production de composés dans lesquels $R^2$ représente un atome d'hydrogène ou un groupe alkyle en $C_1$-$C_3$.

**4.** Procédé selon l'une quelconque des revendications 1 à 3, pour la production de composés dans lesquels $R^3$ et $R^4$ représentent chacun, indépendamment, un atome d'hydrogène ou un groupe alkyle en $C_1$-$C_3$.

**5.** Procédé selon l'une quelconque des revendications 1 à 4, pour la production de composés dans lesquels $R^5$, $R^6$ et $R^7$ représentent chacun un atome d'hydrogène ou un groupe alkyle en $C_1$-$C_3$.

**6.** Procédé selon l'une quelconque des revendications 1 à 4, pour la production de composés dans lesquels $R^5$ et $R^6$ représentent tous deux des atomes d'hydrogène et $R^7$ représente un groupe alkyle en $C_1$-$C_6$, cycloalkyle en $C_3$-$C_7$, alcényle en $C_3$-$C_6$ ou phényl(alkyle en $C_1$-$C_3$).

**7.** Procédé selon l'une quelconque des revendications 1 à 6, pour la production de composés dans lesquels Q représente un atome d'hydrogène, un atome d'halogène ou un groupe hydroxy, alcoxy en $C_1$-$C_3$ ou alkyle en $C_1$-$C_3$.

**8.** Procédé selon la revendication 1, pour la production de composés dans lesquels $R^1$ représente un groupe alkyle en $C_1$-$C_3$, N,N-di(alkyl en $C_1$-$C_3$)carboxamido ou (alcoxy en $C_1$-$C_3$)-carbonyle; $R^2$ représente un atome d'hydrogène; $R^3$ et $R^4$ représentent tous deux des atomes d'hydrogène; $R^5$ et $R^6$ représentent chacun un atome d'hydrogène; $R^7$ représente un atome d'hydrogène ou un groupe alkyle en $C_1$-$C_3$; et Q représente un atome d'hydrogène ou d'halogène, ou un groupe alkyle en $C_1$-$C_3$.

**9.** Procédé selon la revendication 1, pour la production de la 3-(5-méthyl-1H-imidazole-4-yl)-1-(1,7-diméthyl-1H-indole-3-yl)propanone et de ses sels et solvates physiologiquement acceptables.

**Patentansprüche**
**Patentansprüche für folgende Vertragsstaaten : AT, BE, CH, DE, FR, GB, IT, LI, LU, NL, SE**

**1.** Verbindungen der allgemeinen Formel (I):

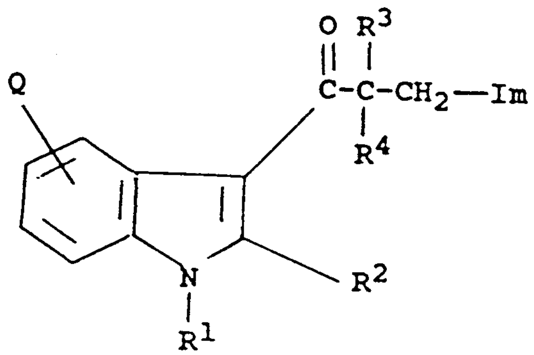

(I)

worin

Im eine Imidazolylgruppe der Formel:

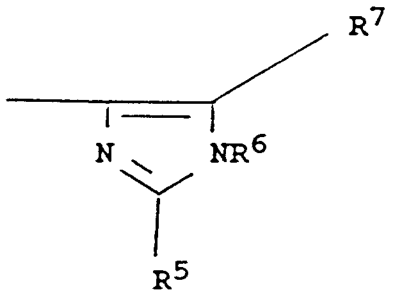

oder

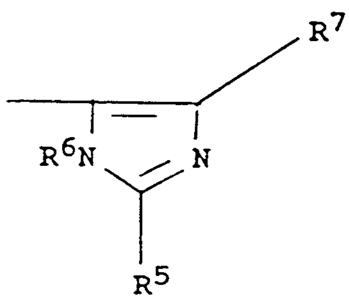

bedeutet;

$R^1$ ein Wasserstoffatom oder eine Gruppe, ausgewählt unter $C_{1-6}$-Alkyl, $C_{3-6}$-Alkenyl, $C_{3-10}$-Alkinyl, $C_{3-7}$-Cycloalkyl, $C_{3-7}$-Cycloalkyl-$C_{1-4}$-alkyl, Phenyl, Phenyl-$C_{1-3}$-alkyl, $-CO_2R^8$, $-COR^8$, $-CONR^8R^9$ oder $-SO_2R^8$ (worin $R^8$ und $R^9$, die gleich oder unterschiedlich sein können, je ein Wasserstoffatom, eine $C_{1-6}$-Alkyl- oder $C_{3-7}$-Cycloalkylgruppe oder eine Phenyl- oder Phenyl-$C_{1-4}$-alkylgruppe bedeuten, wobei die Phenylgruppe gegebenenfalls durch eine oder mehrere $C_{1-4}$-Alkyl-, $C_{1-4}$-Alkoxy- oder Hydroxygruppen oder Halogenatome substituiert sein kann, mit der Maßgabe, daß $R^8$ nicht ein Wasserstoffatom bedeutet, wenn $R^1$ eine Gruppe $-CO_2R^8$ oder $-SO_2R^8$ bedeutet) bedeutet;

$R^2$ ein Wasserstoffatom oder eine $C_{1-6}$-Alkyl-, $C_{3-6}$-Alkenyl-, $C_{3-7}$-Cycloalkyl-, Phenyl- oder Phenyl-$C_{1-3}$-alkylgruppe bedeutet;

$R^3$ und $R^4$, die gleich oder unterschiedlich sein können, je ein Wasserstoffatom oder eine $C_{1-6}$-Alkylgruppe bedeuten;

eine der Gruppen, die durch $R^5$, $R^6$ und $R^7$ dargestellt wird, ein Wasserstoffatom oder eine $C_{1-6}$-Alkyl-, $C_{3-7}$-Cycloalkyl-, $C_{3-6}$-Alkenyl-, Phenyl- oder Phenyl-$C_{1-3}$-alkylgruppe bedeutet und jede der beiden anderen Gruppen, die gleich oder unterschiedlich sein können, ein Wasserstoffatom oder eine $C_{1-6}$-Alkylgruppe bedeuten;

Q ein Wasserstoffatom oder ein Halogenatom oder eine Hydroxy-, $C_{1-4}$-Alkoxy-, Phenyl-$C_{1-3}$-alkoxy- oder $C_{1-6}$-Alkylgruppe oder eine Gruppe $-NR^{10}R^{11}$ oder $-CONR^{10}R^{11}$ bedeutet (worin $R^{10}$ und $R^{11}$, die gleich oder unterschiedlich sein können, je ein Wasserstoffatom oder eine $C_{1-4}$-Alkyl-oder $C_{3-4}$-Alkenylgruppe bedeuten oder zusammen mit dem Stickstoffatom, an das sie gebunden sind, einen gesättigten 5- bis 7gliedrigen Ring bilden);

und mit der Maßgabe, daß, wenn Q ein Wasserstoffatom bedeutet, $R^1$ $-CO_2R^8$, $-COR^8$, $-CONR^8R^9$ oder $-SO_2R^8$ bedeutet; und die physiologisch annehmbaren Salze und Solvate davon.

2. Verbindungen nach Anspruch 1, worin $R^1$ ein Wasserstoffatom oder eine $C_{1-3}$-Alkyl-, $C_{3-4}$-Alkenyl-, $C_{3-4}$-Alkinyl-, $C_{5-6}$-Cycloalkyl-, Benzyl-, N,N-Di-$C_{1-3}$-alkylcarboxamido- oder $C_{1-3}$-Alkoxycarbonylgruppe bedeutet.

3. Verbindungen nach Anspruch 1 oder 2, worin $R^2$ ein Wasserstoffatom oder eine $C_{1-3}$-Alkylgruppe bedeutet.

4. Verbindungen nach einem der Ansprüche 1 bis 3, worin $R^3$ und $R^4$ je unabhängig ein Wasserstoffatom oder eine $C_{1-3}$-Alkylgruppe bedeuten.

5. Verbindungen nach einem der Ansprüche 1 bis 4, worin $R^5$, $R^6$ und $R^7$ je ein Wasserstoffatom oder eine $C_{1-3}$-Alkylgruppe bedeuten.

6. Verbindungen nach einem der Ansprüche 1 bis 4, worin $R^5$ und $R^6$ beide Wasserstoffatome bedeuten und $R^7$ eine $C_{1-6}$-Alkyl-, $C_{3-7}$-Cycloalkyl-, $C_{3-6}$-Alkenyl- oder Phenyl-$C_{1-3}$-alkylgruppe bedeutet.

7. Verbindungen nach einem der Ansprüche 1 bis 6, worin Q ein Wasserstoffatom, ein Halogenatom oder eine Hydroxy-, $C_{1-3}$-Alkoxy- oder $C_{1-3}$-Alkylgruppe bedeutet.

8. Verbindungen nach Anspruch 1, worin $R^1$ eine $C_{1-3}$-Alkyl-, N,N-Di-$C_{1-3}$-alkylcarboxamido- oder $C_{1-3}$-Alkoxycarbonylgruppe bedeutet; $R^2$ ein Wasserstoffatom bedeutet; $R^3$ und $R^4$ beide Wasserstoffatome bedeuten; $R^5$ und $R^6$ je ein Wasserstoffatom bedeuten; $R^7$ ein Wasserstoffatom oder eine $C_{1-3}$-Alkylgruppe bedeutet; und Q ein Wasserstoffatom oder ein Halogenatom oder eine $C_{1-3}$-Alkylgruppe bedeutet.

9. Verbindung nach Anspruch 1, nämlich 3-(5-Methyl-1H-imidazol-4-yl)-1-(1,7-dimethyl-1H-indol-3-yl)-propanon, oder ein physiologisch annehmbares Salz oder Solvat davon.

10. Verfahren zur Herstellung einer Verbindung der allgemeinen Formel (I) nach einem der Ansprüche 1 bis 9 oder eines physiologisch annehmbaren Salzes oder Solvats davon, dadurch **gekennzeichnet,** daß man

(A) eine Verbindung der allgemeinen Formel (II):

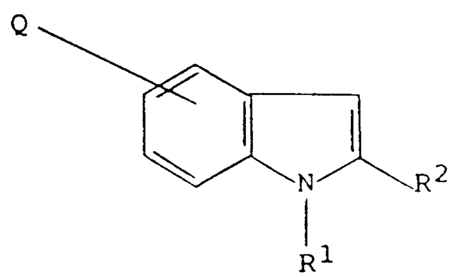

(II)

oder ein geschütztes Derivat davon mit einem Acylierungsderivat einer Säure der allgemeinen Formel (III):

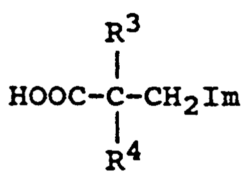

**(III)**

oder einem Salz oder geschützten Derivat davon umsetzt, anschließend gegebenenfalls irgendwelche vorhandenen Schutzgruppen entfernt; oder

(B) für die Herstellung einer Verbindung der Formel (I), worin $R^1$ die Gruppe $-CO_2R^8$, $-COR^8$, $-COR^8R^9$ oder $-SO_2R^8$ bedeutet, je nach Bedarf eine Verbindung der Formel (IV):

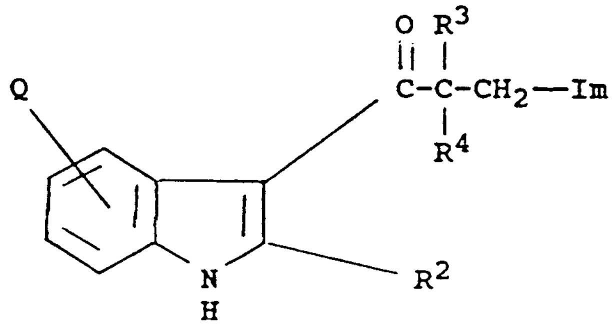

(IV)

oder ein geschütztes Derivat davon acyliert oder sulfonyliert und gegebenenfalls anschließend irgendwelche vorhandenen Schutzgruppen entfernt; oder

(C) eine Verbindung der Formel (I) oder ein Salz oder geschütztes Derivat davon in eine andere Verbindung der Formel (I) unter Verwendung an sich bekannter Verfahren umwandelt; oder

(D) eine oder mehrere Schutzgruppe(n) aus einer geschützten Form einer Verbindung der Formel (I) entfernt;

und, wenn die Verbindung der Formel (I) als ein Gemisch von Enantiomeren erhalten wird, gegebenenfalls das Gemisch unter Bildung des gewünschten Enantiomeren spaltet;

und/oder, wenn die Verbindung der Formel (I) in Form einer freien Base erhalten wird, gegebenenfalls die freie Base in ein Salz überführt.

**11.** Pharmazeutisches Präparat, dadurch **gekennzeichnet,** daß es mindestens eine Verbindung der allgemeinen Formel (I), definiert in einem der Ansprüche 1 bis 9, oder ein physiologisch annehmbares Salz oder Solvat davon zusammen mit mindestens einem physiologisch annehmbaren Träger oder Verdünnungsstoff enthält.

**12.** Verbindung der allgemeinen Formel (I) nach Anspruch 1 für die Verwendung als aktives therapeutisches Mittel.

**Patentansprüche für folgende Vertragsstaaten : ES, GR**

**1.** Verfahren zur Herstellung von Verbindungen der allgemeinen Formel (I):

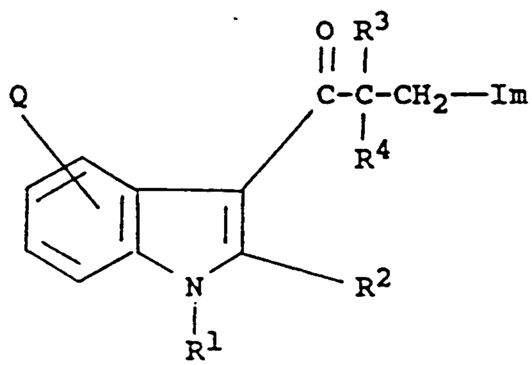

(I)

worin

Im eine Imidazolylgruppe der Formel:

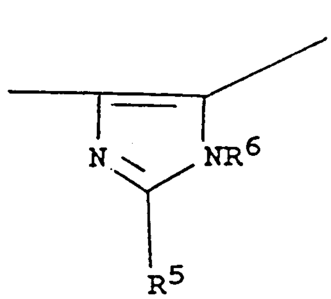

oder

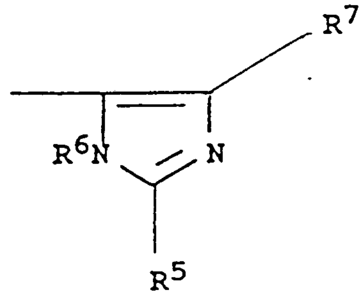

bedeutet;

$R^1$ ein Wasserstoffatom oder eine Gruppe, ausgewählt unter $C_{1-6}$-Alkyl, $C_{3-6}$-Alkenyl, $C_{3-10}$-Alkinyl, $C_{3-7}$-Cycloalkyl, $C_{3-7}$-Cycloalkyl-$C_{1-4}$-alkyl, Phenyl, Phenyl-$C_{1-3}$-alkyl, $-CO_2R^8$, $-COR^8$, $-CONR^8R^9$ oder $-SO_2R^8$ (worin $R^8$ und $R^9$, die gleich oder unterschiedlich sein können, je ein Wasserstoffatom, eine $C_{1-6}$-Alkyl- oder $C_{3-7}$-Cycloalkylgruppe oder eine Phenyl- oder Phenyl-$C_{1-4}$-alkylgruppe bedeuten, wobei die Phenylgruppe gegebenenfalls durch eine oder mehrere $C_{1-4}$-Alkyl-, $C_{1-4}$-Alkoxy- oder Hydroxygruppen oder Halogenatome substituiert sein kann, mit der Maßgabe, daß $R^8$ nicht ein Wasserstoffatom bedeutet, wenn $R^1$ eine Gruppe $-CO_2R^8$ oder $-SO_2R^8$ bedeutet) bedeutet;

$R^2$ ein Wasserstoffatom oder eine $C_{1-6}$-Alkyl-, $C_{3-6}$-Alkenyl-, $C_{3-7}$-Cycloalkyl-, Phenyl- oder Phenyl-$C_{1-3}$-alkylgruppe bedeutet;

$R^3$ und $R^4$, die gleich oder unterschiedlich sein können, je ein Wasserstoffatom oder eine $C_{1-6}$-Alkylgruppe bedeuten;

eine der Gruppen, die durch $R^5$, $R^6$ und $R^7$ dargestellt wird, ein Wasserstoffatom oder eine $C_{1-6}$-Alkyl-, $C_{3-7}$-Cycloalkyl-, $C_{3-6}$-Alkenyl-, Phenyl- oder Phenyl-$C_{1-3}$-alkylgruppe bedeutet und jede der beiden anderen Gruppen, die gleich oder unterschiedlich sein können, ein Wasserstoffatom oder eine $C_{1-6}$-Alkylgruppe bedeuten;

Q ein Wasserstoffatom oder ein Halogenatom oder eine Hydroxy-, $C_{1-4}$-Alkoxy-, Phenyl-$C_{1-3}$-alkoxy- oder $C_{1-6}$-Alkylgruppe oder eine Gruppe $-NR^{10}R^{11}$ oder $-CONR^{10}R^{11}$ bedeutet (worin $R^{10}$ und $R^{11}$, die gleich oder unterschiedlich sein können, je ein Wasserstoffatom oder eine $C_{1-4}$-Alkyl-oder $C_{3-4}$-Alkenylgruppe bedeuten oder zusammen mit dem Stickstoffatom, an das sie gebunden sind, einen gesättigten 5- bis 7gliedrigen Ring bilden);

und mit der Maßgabe, daß, wenn Q ein Wasserstoffatom bedeutet, $R^1$ $-CO_2R^8$, $-COR^8$, $-CONR^8R^9$ oder $-SO_2R^8$ bedeutet; und die physiologisch annehmbaren Salze und Solvate davon, dadurch **gekennzeichnet,** daß man

(A) eine Verbindung der allgemeinen Formel (II):

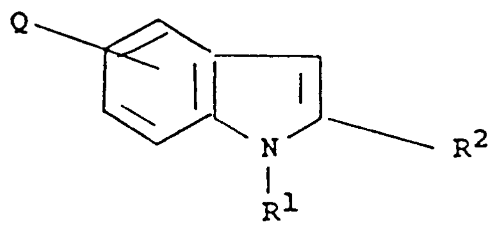

(II)

oder ein geschütztes Derivat davon mit einem Acylierungsderivat einer Säure der allgemeinen Formel (III):

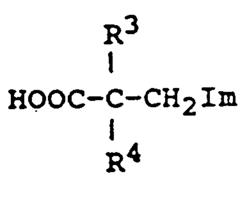

(III)

oder einem Salz oder geschützten Derivat davon umsetzt, anschließend gegebenenfalls irgendwelche vorhandenen Schutzgruppen entfernt; oder

(B) für die Herstellung einer Verbindung der Formel (I), worin $R^1$ die Gruppe $-CO_2R^8$, $-COR^8$, $-COR^8R^9$ oder $-SO_2R^8$ bedeutet, je nach Bedarf eine Verbindung der Formel (IV):

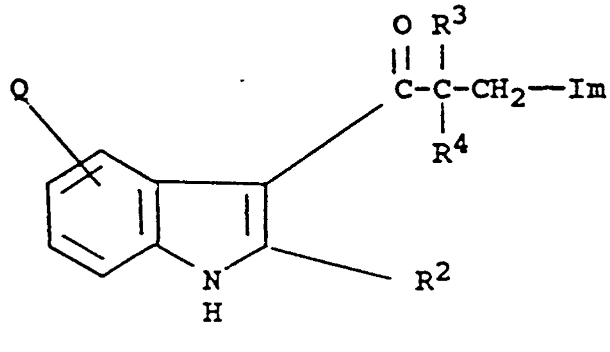

(IV)

oder ein geschütztes Derivat davon acyliert oder sulfonyliert und gegebenenfalls anschließend irgendwelche vorhandenen Schutzgruppen entfernt; oder

(C) eine Verbindung der Formel (I) oder ein Salz oder geschütztes Derivat davon in eine andere Verbindung der Formel (I) unter Verwendung an sich bekannter Verfahren umwandelt; oder

(D) eine oder mehrere Schutzgruppe(n) aus einer geschützten Form einer Verbindung der Formel (I) entfernt;

und, wenn die Verbindung der Formel (I) als ein Gemisch von Enantiomeren erhalten wird, gegebenenfalls das Gemisch unter Bildung des gewünschten Enantiomeren spaltet;

und/oder, wenn die Verbindung der Formel (I) in Form einer freien Base erhalten wird, gegebenenfalls die freie Base in ein Salz überführt.

**2.** Verfahren nach Anspruch 1, dadurch **gekennzeichnet,** daß Verbindungen hergestellt werden, worin $R^1$ ein Wasserstoffatom oder eine $C_{1-3}$-Alkyl-, $C_{3-4}$-Alkenyl-, $C_{3-4}$-Alkinyl-, $C_{5-6}$-Cycloalkyl-, Benzyl-, N,N-Di-$C_{1-3}$-alkylcarboxamido- oder $C_{1-3}$-Alkoxycarbonylgruppe bedeutet.

**3.** Verfahren nach Anspruch 1 oder 2, dadurch **gekennzeichnet,** daß Verbindungen hergestellt werden, worin $R^2$ ein Wasserstoffatom oder eine $C_{1-3}$-Alkylgruppe bedeutet.

**4.** Verfahren nach einem der Ansprüche 1 bis 3, dadurch **gekennzeichnet,** daß Verbindungen hergestellt werden, worin $R^3$ und $R^4$ je unabhängig ein Wasserstoffatom oder eine $C_{1-3}$-Alkylgruppe bedeuten.

**5.** Verfahren nach einem der Ansprüche 1 bis 4, dadurch **gekennzeichnet,** daß Verbindungen hergestellt werden, worin $R^5$, $R^6$ und $R^7$ je ein Wasserstoffatom oder eine $C_{1-3}$-Alkylgruppe bedeuten.

**6.** Verfahren nach einem der Ansprüche 1 bis 4, dadurch **gekennzeichnet,** daß Verbindungen hergestellt werden, worin $R^5$ und $R^6$ beide Wasserstoffatome bedeuten und $R^7$ eine $C_{1-6}$-Alkyl-, $C_{3-7}$-Cycloalkyl-, $C_{3-6}$-Alkenyl- oder Phenyl-$C_{1-3}$-alkylgruppe bedeutet.

**7.** Verfahren nach einem der Ansprüche 1 bis 6 , dadurch **gekennzeichnet,** daß Verbindungen hergestellt werden, worin Q ein Wasserstoffatom, ein Halogenatom oder eine Hydroxy-, $C_{1-3}$-Alkoxy- oder $C_{1-3}$-Alkylgruppe bedeutet.

**8.** Verfahren nach Anspruch 1, dadurch **gekennzeichnet,** daß Verbindungen hergestellt werden, worin $R^1$ eine $C_{1-3}$-Alkyl-, N,N-Di-$C_{1-3}$-alkylcarboxamido-oder $C_{1-3}$-Alkoxycarbonylgruppe bedeutet; $R^2$ ein Wasserstoffatom bedeutet; $R^3$ und $R^4$ beide Wasserstoffatome bedeuten; $R^5$ und $R^6$ je ein Wasserstoffatom bedeuten; $R^7$ ein Wasserstoffatom oder eine $C_{1-3}$-Alkylgruppe bedeutet; und Q ein Wasserstoffatom oder ein Halogenatom oder eine $C_{1-3}$-Alkylgruppe bedeutet.

**9.** Verfahren nach Anspruch 1, dadurch **gekennzeichnet,** daß 3-(5-Methyl-1H-imidazol-4-yl)-1-(1,7-dimethyl-1H-indol-3-yl)-propanon und die physiologisch annehmbaren Salze und Solvat davon herge-

stellt werden.